(19)

Europäisches
Patentamt
European
Patent Office
Office européen
des brevets

(11) **EP 2 433 619 A1**

(12) **EUROPEAN PATENT APPLICATION**

(43) Date of publication:
**28.03.2012 Bulletin 2012/13**

(51) Int Cl.:
**A61K 9/127** (2006.01)   **A61K 31/575** (2006.01)
**A61P 35/00** (2006.01)

(21) Application number: **10010482.7**

(22) Date of filing: **24.09.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Intsel Chimos
92213 Saint-Cloud Cedex (FR)**

(72) Inventor: **The designation of the inventor has not
yet been filed**

(74) Representative: **Burtin, Jean-François
Cabinet Gefib
c/o Sté Delpharm
Immeuble Le Poversy Bât. B
6-8 rue du 4 septembre
92130 Issy-les-Moulineaux (FR)**

(54) **Novel antitumoral agent and its therapeutic uses**

(57)   This invention relates to the field of the needs of the life and namely to the field of therapy.

It has as the subject matter the use of 7β-hydroxycholesteryl 3β-oleate ester (7-β-OHCOE) in the form of a lipid dispersion directly in the tumours in the forms of liposomes in patients suffering forms anaplastic astrocytomas or glioblastomas.

From a short clinical assay on patients at a dosis ranging from 0,05 to 3,2 mg per patient a significant reduction of this total tumoral volume has been observed.

EP 2 433 619 A1

**Description**

This invention relates to the field of needs of the life and namely to the field of therapy.

This invention more precisely relates to the field of oncology and particularly to the treatment of primary brain tumours.

Specifically this invention refers to the use of 7-β-hydroxy cholesteryl-3-β-oleate-ester (7-β-OHCOE) in the form of a lipid dispersion directly in the tumour. Preferably this preparation is in the form of neutral liposomes or in the form of charged (negative) liposomes. The preparation may also be a solution or a suspension in a lipidic carrier such as medium chain triglyceride or a mixture of various medium sized triglycerides.

The carrier may also be made out of ceramide or pseudo ceramide derived from natural vegetal or animal oils or from synthetic ceramides incorporating fatty acids extracted from natural resources.

A prospective open study about tolerance and safety of 7-β-OHCOE with a pharmaceutical presentation of liposomes and administered into the intra-cerebral tumoral site in patients suffering from anaplastic astrocytoma or glioblastoma with an at least partially kystic component has been performed. This phase I study was a prerequisite for a 7-β-OHCOE efficacy study in the treatment of anaplastic astrocytomas and glioblastomas.

This new therapeutic approach by intratumour injection of 7-β-OHCOE formulated as liposomes has shown efficacy from the first injection in animal models. Thus, with patients who have relapsed in spite of current treatments, an early tumoral response may be expected. Gliomas (or glioblastomas) are primary brain tumours that begin in brain tissue. They are classified by the type of tissue from which they originate. The most common brain tumours are gliomas, which begin in the glial (supportive) tissue. Due to their localisation, gliomas represent a potentially debilitating and life threatening condition. Patients affected by gliomas can suffer from neurological complications, depending on the site of intra-cerebral development of the tumour. (EMA, public summaries of orphan designations in gliomas)

High-grade malignant gliomas (grades III and IV) are serious diseases affecting on the short term the vital prognosis. The average survival is about 1 year (Stupp 2005, Brada 2000).

Glioblastoma multiform (GBM) is the highest degree of malignancy of glial tumours (grade IV of the World Health Organization) with a bleak short term prognosis: 9 to 12 months median survival and 2-year survival rates between8 and 12 % (EPAR, Temozolomide®) GBM is the most common and most aggressive of the primary brain tumours in adults. It represents 15% to 20 % of all brain tumours and about 50 % of all gliomas. It is highly malignant, infiltrates the brain extensively, and at times may become enormous before turning symptomatic.

The current World Health Organization (WHO) classification of primary brain tumours lists GMB as a grade IV astrocytoma. GBM is slightly more common in men than in women; the male to female ratio is 3:2. While GBM occurs in all age groups, its incidence is increasing in elderly patients. A true increase in incidence of primary brain tumours exists, which cannot be explained by the aging population, better imaging techniques, or earlier detection at surgery. GBM is an anaplastic, highly cellular tumour with poorly differentiated, round, or pleomorphic cells, occasional multinucleated cells, nuclear atypia, and anaplasia. Under the modified WHO classification, GBM differs from anaplastic astrocytomas (AA) by the presence of necrosis under the microscope. (EPAR, Temodal®)

Known risk factors for primary brain tumours include exposure to therapeutic ionising radiation, employment in synthetic rubber manufacturing, petroleum refining or production work, and exposure to vinyl chloride or to pesticides. Therapeutic ionising radiation is a strong risk for brain tumours (Wrensch 2002).

Second primary brain malignancies also occurred more frequently than expected, especially among patients treated with radiotherapy.

Slightly higher relative risk has been associated with passive exposure of the child or mother to a smoking father. The results from exposure to passive smoking by the father suggested a slightly increased relative risk of 1.2 based on ten studies (Wrensch 2002).

Exposure to filter cigarettes, diagnostic ionising radiation, residential electromagnetic fields, formaldehyde, and cell phone use are not proven risk factors (Wrensch 2002).

Recently it has been published a meta-analysis based on 2 cohorts and 16 case-control studies on the use of mobile phones for 10 years (Hardell 2007). The results from this analysis give a consistent pattern of an increased risk for glioma and acoustic neuroma.

The risk is higher for ipsilateral exposure. From these studies, however, it is not clear at what stage microwaves may act in carcinogenesis.

Familial aggregation of brain tumours, gliomas in particular, has been reported in 5% of cases (Wrensch 1997). In many cases, a hereditary syndrome cannot be identified in brain tumour families. Sib pairs with glioma have often been observed (Grossman 1999). Two segregation analyses have been performed on consecutive patients with glioma and their close relatives. One study indicated that an autosomal recessive gene plays a role in cancer aggregation in glioma families (Malmer 2001), whereas the other suggested a multifactorial cause (de Andrade 2001).

If the risk in siblings is high, an autosomal recessive gene or an environmental exposure may be suspected. To study the effect of environmental versus genetic effects, Malmer et al. (Malmer 2003) compared the risk in first-degree relatives (FDR; siblings, parents, and children) who developed the same site primary brain tumour, with the risk in spouses (husbands and wives) of primary brain tumours patients. No increase in risks of any specific type of brain tumour was found in the cohort of spouses. However, in the cohort of first degree relatives, the overall risk of primary brain tumour was significantly increased, by 2 or 3 fold for subjects with the same histopathology as the probands; this indicates that the familial aggregation of brain tumours is of genetic origin.

These compositions are intended for the local treatment of high grade indignant gliomas (grades III and IV).

## Medical plausibility

The prognosis for patients with glioma, and in particular those with multiforme glioblastoma (GBM) (in the literature often also referred to as glioma grade IV), remains dire, with a median survival of only 25 weeks.

At present, in particular with regards to the non-surgical forms, there is no form of treatment which significantly prolongs the survival of the patients.

In view of this, the product, an ester of 7-β-hydroxy cholesterol, was initially developed by the Laboratoire de Neurobiologie Moléculaire des Interactions Cellulaires, UPR 416 CNRS, Strasbourg, France.

An ester of the 7 beta-hydroxy-cholesterol, belonging to the family of oxysterols, the 7 β-hydroxy-cholesteryl-3-β-oleate-ester (7-β-OHCOE) possesses an anti-proliferative effect on cells in the active multiplication phase, in particular on C6 cell cultures obtained from glioblastomas in rats.

The effect of 7-β-hydroxy cholesteryl-3-β-oleate-ester on rat brain C6 glioblastoma cells was studied (Werthle M & All). The injection of liposomes without oxysterol had no effect on tumor growth, whereas injection of liposomes containing 7-β-hydroxy cholesteryl-3-β-oleateester gave rise to a marked decrease in tumor volume.

In addition, in rats having undergone an intracerebral implantation of C6 glial cells, an intratumoral injection of liposomes (double-stratum lipidic vesicles) containing the 7-β-OHCOE has resulted in a significant regression of tumor growth in comparison with animals treated with liposomes only (control group).

Post-treatment toxicological studies in the treated rats and mice (previous studies on naturally ill mice: mice from hybrid CDF1 strain (Balb/c x DBA2), have shown no noxious effects, 7-β-OHCOE is metabolized to cholesterol within the cerebral parenchyma. It has thus been proposed to further study the efficacy of liposome-formulated 7-β-OHCOE through stereotactic intratumoral injection in glioblastomas in humans.

## Justification of the life-threatening or debilitating nature of the condition

Morbidity is depending on the tumour location, progression, and pressure effects. The overall prognosis for GBM has changed little in the past 2 decades, despite major improvements in neuroimaging, neurosurgery, radiation treatment techniques, supportive care and new chemotherapy agents and regimens. Prognosis for GBM remains poor, the median survival is 9 to 12 months, the 2 year survival rates are between 8% and 12% (EPAR, Temodal®). Taking into account the current prognosis associated with the usual management of the disease, there is a significant therapeutic public health need.

The first line malignant high-grade gliomas treatment is surgical resection, followed by an external radiotherapy.

Radiotherapy randomized trials have consistently shown an improved survival rate compared to results of surgical treatment alone (Walker et coll, 1980). In some cases, a systemic chemotherapy (including Nitrosourea, Procarbazine, Carboplatine or Temozolomide®) is used in addition to radiotherapy; however, none of these treatments had proved efficient in double blind controlled randomized trials in patients suffering of primary malignant glioma (Chang et Padros, 1995; Culver et coll, 1992 ; Fine et coll, 1993;Hildebrand et coll, 1997; Lesser et Grossman, 1994; Prados et Levin, 2000; Walker et coll, 1980).

Following the first results which have been obtained by the applicant with 7-β-hydroxycholesteryl-3-β-oleate-ester it is observed that they have a greater efficacy on the daily comfort of life of the patients and also on the possibility to cure them without any undesirable effect.

## Details on any existing diagnosis, prevention or treatment methods

The standard treatment of malignant gliomas includes maximum surgical resection, when feasible, followed by partial brain radiotherapy. Radiotherapy can be combined or followed by chemotherapy. Although the clinical benefit of chemotherapy is only small, chemotherapy agents are used for the treatment of GBM: Cytotoxic agents most commonly applied for chemotherapy are nitrosourea-based regimens such as BCNU (carmustine) and procarbazine, futhermore, vinca alkaloids, platinum compounds, cyclophosphamide, methotrexate, temozolomide (EPAR,Temozolomide) are used.

The first line malignant high-grade gliomas treatment is surgical resection, followed by an external radiotherapy.

Radiotherapy randomized trials have consistently shown an improved survival rate compared to results of surgical treatment alone (Walker et coll, 1980). Recent OERT randomized trial shown that radiotherapy with concomittent temozolomide improved survival rates in patients with gross total resection (Stupp 2007). At recurrence, a systemic chemotherapy (including nitrosourea, procarbazine, carboplatine or temozolomide) is used in addition to radiotherapy ; however, none of these treatments had proved efficient in double blind controlled randomized trials in patients suffering of primary malignant glioma (Chang and Prados, 1995 ; Culver and coll, 1992 ; Fine and coll, 1993 ; Hildebrand and coll, 1997 ; Lesser and Graossman, 1994 ; Prados and Levin, 2000 ; Walker and coll, 1980).

Recently a randomized trial showed that the combination of bevacizumab and irinotecan could lead to significant tumoral response. (Vredenburgh JJ, 2007)

In spite of some improvements due to new intratumoral administration forms of antineoplastic agents or protocols combining chemotherapy and radiotherapy, the anaplastic astrocytomas and glioblastomas prognosis remains dire with a median survival of about 25 weeks (Rainov, NG. 2000, Croteau, D. and Mikklesen, T. 2001, Behin, A. & coll., 2003, Stupp, R. & coll. 2004). The most efficient treatments only slightly prolong the patients survival, notably in case of non surgical forms.

From the EUROCARE study and the SEER programme (Capocaccia 2003; SEER 2007) survival for GMB is available from population-based cancer registries. Prognosis for GBM is very poor. Relative survival for adults diagnosed with GBM was, in European and US populations, less than 30 % at one year, 5% at three years, and 3% at five years, with no difference between men and women. Five-year relative survival decreased markedly with age from 13% to less than 1% from the youngest (15-45 years) to the oldest age group of patients (75 years and over). Data from the more recent randomised phase III trials and meta-analysis give substantially better survival rates than population-based registries, showing a two years survival rates of 13-26.5 % (Stewart 2002; Stupp 2005). Data from clinical trials may due in part to improvement in therapeutic options, but may also reflect survival in selected patients with more favourable prognostic factors.

The ester of 7-β-hydroxy-cholesterol, the 7-β-hydroxy cholesteryl-3-β-oleate-ester (7-β-OHCOE) belongs to the oxysterols. Several published studies show that oxysterols have an anti-proliferation activity on cells in active multiplication phase, notably cells of nervous origin. This data are confirmed by studies on rats having been implanted with C6 proliferative glial cells. The intratumour injection of liposomes containing 7-β-OHCOE is followed by a spectacular regression of tumour growth compared to animals treated with control liposomes.

The most remarkable property of oxysterols is their lethal property for cells with a high rate of proliferation in models of mammal cells in *vitro* (Smith, L.L., 1989) and in *vivo* (Rong, S. 1985, Iversen, O.H. 1986, Christ, M, 1991). Among compounds of the OH-CH family, one of the most cytotoxic is 7-β-OH cholesterol which can be found in extracts of Bombix cum Botyte, a silk worm infested by a fungus that has a lethal action on transformed or tumoral cells (Nagano H 1977, Hietter H 1986, Mersel M 1984, Nordmann, P 1989). On the other hand, some differentiated cells with little proliferation, are not affected by this substance.

At this date, it is thought that the lethal effect of oxysterols is the result, during the steps of transcription and post transcription, of several overlapping occurrences (Edward, P.A 1999), mainly these three: inhibition of cholesterogenesis, interference in the traffic of cell messengers, destabilization of the architecture and dynamics of cell membranes.

Several studies show the lethal effect of 7-β-OH-CH on tumour astrocyte lines, transformed spontaneously, obtained from primary cultures of astrocytes of newborn rat brain. The cytotoxicity, expressed by retractation, loss of adhesion and cell death, is correlated by the esterification rate of 7-β-OH-CH into 7-β-OHCOE by fatty acids (Kupferberg, A & coll. 1989, Behr, P & coll. 1992). The treatment of tumoral cells by oxysterol leads to the blockade of their cell division.

About the modes of action, the set of in *vitro* studies suggest that the action modes differ between the esterified 7-β-OH-CH *in situ* and 7-β-OHCOE. For 7-β-OH-CH *in situ* the oxysterol probably acts upon the endoplasmic reticulum at the level of isopentenyl-Pyrophosphate (PP) => farnesyl-PP step, leading to a reduced farnesyl-Ras and a system dolichyl-PP-oligosaccharide => surface N-glycoproteins disturbed (Rakotoarivelo, C. 1998, Kupferberg, A 1992 et 1997). On the other hand, the 7 β-OHCOE, located in the plasmic membrane, probably lowers the synthesis rate of cholesterol, by way of slowing down the cytokinesis and reducing by feed back Ras/Raf expression (Janowski, B.A 2001).

This invention essentially relates to a process for producing neutral or charged liposomes containing as the active ingredient, 7-β-hydroxy cholesteryl-3-β-oleate (named as oxysterol) intended to obtain a preparation to be directly injected in a carrier, into malignant gliomas.

More precisely the said liposomes are obtained in dissolving 7-β-hydroxy cholesteryl-3-β-oleate in a mixture of dichloromethane and anhydrous ethanol in which previously soya lecithin and sodium cholesterylsulphate have been dissolved.

The subsequent step is the evaporation step in a rotatory evaporator under reduced pressure of the mixture of solvents to obtain a lipidic film on the walls of the container.

The lipidic layer is taken up with a phosphate buffer which allows a pH value of the mixture of about 6,8. This production needs a prolonged heating time of the mixture at moderate temperatures ranging from 50 to 80 ° C and mainly from 60 - 65 °C. The technical problem of importance is to obtain liposomes of homogeneous sizes, for example from 100 to 600 nm and mainly from 300 to 400 nm.

After complete mixing with the buffer and heating up to 60 °C +/- 5°C for a set of time extending for at least 3 hours under reduced pressure a whitish to greyish suspension is obtained.

This suspension is sterilized by heating at 110 °C for 20 minutes or by filtering on a filter of 0,22 μm of pore size under a laminar flux. The sterilized suspension is divided into glasses of 10 ml content.

The sterilized suspension is intended to be used as such or further diluted with a sterile medium, for example a lipidic medium such as Intralipid. The suspension of liposomes or the diluted suspension may be injected into the brain tumour. The content of active ingredient ranges from 1.4070 to 1.83mg/g of liposomes, and preferably from 1,60 to 1,70 mg/g liposomes.

Experimental Part

Exemple I : Preparation of a batch of 100 to 250 g of liposomes of Oxysterol.

1. Composition:

Composition of the saline solution buffered with phosphate at pH 6,8 (Ph. Eur. Réf. 4003200)

| Raw Materials | Composition by weight (g) | Composition in percentage (w/v) |
|---|---|---|
| Monopotassium phosphate | 0,50 | 0,1 |
| Dipotassium phosphate | 1,00 | 0,2 |
| Sodium chloride | 4,25 | 0,85 |
| 0,1 N hydrochloric Acid | qsp pH 6,8 | qsp pH 6,8 |
| Water for injectable solutions | qsp 500 ml | qsp 100 |

Composition of liposomal solution of Oxysterol.

| Raw Materials | Composition by weight (g) | Composition in percent % (w/v) | Composition by unit (mg/flask) |
|---|---|---|---|
| Epikuron 200 | 0,7500 g | 0,7500 | 75 |
| Sodium cholesteryl sulphate | 0,0488 g | 0,0488 | 4,88 |
| 7 β-Hydroxy cholesteryl-3-β oleate | 0,1660 g | 0,1660 | 16,6 |
| Dichloromethane | 14,0000 g | - | - |
| Anhydrous ethanol | 4,0000 g | - | - |
| Saline solution buffered with phosphate at pH 6.8 | 99,0352 g | 99,0352 | 990,352 |

2. Performance

a. Preparation of the lipidic layer.

Epikuron 200 and sodium cholesterol sulphate are first dissolved in a solution of dichloromethane and ethanol, then a solution of Oxysterol in dichloromethane is added. After complete dissolution under stirring, evaporation of the solvent in a rotatory evaporator at a temperature of about 56 °C, with a speed of rotation of the flask in the rotatory evaporator : 150 - 250 RPM or in a ventilated room at 40°C for 65 hours.

To the dry residue a saline solution of sodium phosphate buffered at 6,8 is added. The whole mixture is heated at 60-65°C, until the size of the thus formed liposomes is homogeneous. The suspension is then sterilized by treating it at 121 °C for 20 minutes.

The sterilized preparation are poured in to flasks of clear glass type I. Volume of pouring = 100 ml.

The size of the various batches may vary from 100 g to 250 g. The performance of the process is exactly the same except that the duration of the evaporation of the solvents may vary from 2 to 5 minutes under limited pressure then 30 minutes of maximal vacuum.

b. Dosage of Oxysterol

A HPLC/UV method has been performed to obtain the dosage of Oxysterol in the intermediate solution and in the finished product.

- Analysis conditions

Pump Agilent HP 1100

Injector Agilent HP 1100

Detector Agilent HP 1100 with photodiode

Recorder - Integrator : Chemistator

- Mode of performance :

Column n° 242 licrospher RP18 5$\mu$m of 250- 4,6 mm

Mobil Phase : acetonitrile 44 %

Isopropanol 54 %

Water 2 %

The mixture is filtered on a Nylon filter 0,45$\mu$m

Outflow : 1ml/min

Detection UV at 10 nm

Injection loop: 50 pl

Temperature of the column 25° C.

Duration of the analysis: 50 mn

Retention time of Oxysterol 15,7 mn

c. Stability of Oxysterol to autoclave sterilisation

These tests have been performed on the solutions and on the finished product.

A solution of 5 mg Oxysterol in 5 ml of a mixture Acetonitrile / isopropanol (44/56 v/v) is prepared then sterilized in the autoclave for 20 min at 121°C. The solution is analysed according to a spectrophotometric method of oxysterol before and after autoclaving. The solution of oxysterol is subjected to a second autoclaving for 30minutes at 121°C. The areas of the peaks of oxysterol are determined and the yield after autoclaving is calculated in the following manner

$$\text{Yield}: \frac{\text{Area of oxysterol after autoclaving}}{\text{Area of oxysterol before autoclaving}} \times 100$$

The active ingredient seems to be stable to the autoclaving. Not any colouration of the solution is perceptible after autoclaving.

| Solution | Area oxysterol before autoclaving | Area oxysterol after autoclaving | Yield of autoclaving |
|---|---|---|---|
| 1 autoclaving | 5506,5 | 5517,4 | 100,2 % |
| 2 autoclavings | 5666,1 | 5716,3 | 100,9 % |

**[0071]** Thus the solution appears to be stable to autoclaving since no coloration of the solutions is observed after autoclaving.

Active principle in the finished product

**[0072]** A batch has been subjected to a dosage before and after autoclaving 20 minutes at 121 °C, to determine whether oxysterol also resists to autoclaving. Three samples have been analysed :

| Content in oxysterol | PE1 | PE2 | PE3 | Average |
|---|---|---|---|---|
| Before autoclaving | 1,45 mg/g | 1,46 mg/g | 1,45 mg/g | 1,46 mg/g |
| After autoclaving | 1,42 mg/g | 1,41 mg/g | 1,45 mg/g | 1,43 mg/g |

**[0073]** The autoclaving yield is calculated in the following manner

$$\text{Yield autoclaving : } \frac{\text{Content oxysterol after autoclaving}}{\text{Content oxysterol before autoclaving}} \times 100$$

**[0074]** The average yield is of 97,9%. The active ingredient in the finished product is stable after autoclaving. Not any coloration is perceptible after autoclaving

*Exemple II :*

**[0075]** Production for a batch of 300 g of liposomic solution of Oxysterol (7 β-hydroxycholesteryl-3-β-oleate) to determine the critical parameters in the manufacture and in the performance of pre-stability test.

| A. The Raw materials | In percentage |
|---|---|
| 7-β-hydroxy cholesteryl-3-β-oleate | 0,1660 |
| Epikuron 200 | 0,750 |
| Sodium cholesterylsulphate | 0,0488 |
| Dichloromethane | } Mixture of equal masses |
| Anydrous ethanol | |
| Saline solution buffered with sodium phosphate at pH 6,8 | 99,0352% |

B. Composition of the buffer pH 6,8 In percentage (w/v) Monopotassium phosphate 0,1 Dipotassium phosphate 0,2 Sodium chloride 0,85 0,1 N Hydrochloric acid qsp pH 6,8 Water for injectible preparations, enough for 100g

C. Amounts of raw materials for the preparation of a batch of 300g :

**[0076]**

| Raw materials | In g |
|---|---|
| 7-β-hydroxy cholesteryl-3-β-oleate (oxysterol) | 0,498 |
| Epikuron 200 | 2,25 |
| Sodium cholesteryl sulphate | 0,1464 |
| dichloromethane | - |
| Anhydrous ethanol | - |
| Saline solution buffered to pH 6,8 with phosphate | 297,1056 |

D. Process of manufacture and testings during the process

a) the steps
The manufacture is performed along the lines of good practices of manufacture while insuring on the conformity of the raw materials and verifying the cleanness of the materials
b) Weighing of the raw materials
c) Dissolution
In a 500 ml flask they are introduced 2,25g Epikuron 200, 0,1464g of sodium cholesteryl sulphate and 0,498g 7 β-hydroxycholesteryl-3-β-oleate. The dissolution is obtained under magnetic stirring at the water bath at 50-55°C while progressively adding through a burette, 54g of a mixture of dichloromethane/anhydrous ethanol of 14/14 (m/m). After 10 minutes stirring with a magnetic bar, the solution is sonicated for 5 minutes and the absence of particles is verified. Thereafter 3ml dichloromethane are added to dissolve the ultimate particles and one magnetically stirs for about 10 minutes.
d) Obtention of a lipidic film — evaporation of diluting solvents.
The solvents are evaporated at the Rotavapor

- Vacuum at the beginning of evaporation ≈ 100 mbars,
- Temperature of the water bath : at 50 °C +/- 5°C
- Speed of rotation 150 Rpm
- Duration: 15 min

Vacuum during the follow-up:

- Vacuum - 750 mbar
- Temperature of water bath : 50° C. $\pm$ 5
- Speed of rotation: 210 RPM
- Duration: 1 hour

The lipidic film is then put in the oven at 40 °C for 15 hour to eliminate the whole quantities of solvents.
e) Dispersion of the lipidic film
In a gauged flask of 100 ml they are introduced 880 mg water for injectable preparations, 1g monopotassium phosphate, 2g dipotassium phosphate and 8,5g sodium chloride. The mixture is magnetically stirred until complete dissolution (about 3min). The pH value is adjusted to 6,8 using 1mol/1 of hydrochloric acid (introduced amount 1,28g) and the total volume is adjusted to the gauge line with water for injection (introduced amount = 112,9 g). On the lipidic film on the flask walls, they are introduced 297.1056g of the buffer solution at pH 6,8 at 60 °C +/- 5°C
The dispersion of the lipidic film is performed using a magnetic stirring (speed 500 t.min$^{-1}$) at the water bath at 60°C +/- 5° for 3 hours.

Homogeneization through passing into a Microfluidizer M110S

Apparatus: Microfluidizer Model M-1 10S
Interaction Room F12Y 72 $\mu$ ceramic 5568
Pressure 550 bars
Number of passings : 5

| Designation | Batch n°5 |
|---|---|
| N° of the batch | 0510407107A |
| Size of the batch | 250 g |
| Dissolution of the lipidic constituents | Difficulties in dissolving sodium cholesteryl sulphate and Oxysterol |
| Evaporation of the solvents | Temperature 50°C<br>5 minutes limited vacuum<br>30 minutes maximal vacuum storage in the stirring oven 40 °C : 15 hours |
| Dispersion of the lipidic film | Temperature 60 - 65 °C<br>Magnetic stirring for 2 hours |
| Homogenization of the size of liposomes | Pressure at deposit : 550 bars<br>Pressure for deposit homogeneization : 1000 bars<br>Number of passings : 5<br>Piston runs for the emptying of the circuit: 5 moves |
| Sterilization | Filtering is impossible<br>Autoclaving at 121°C for 20 minutes |
| Physical stability | Greyish solution, seemingly due to the microfluidizer<br>Occurrence of a supernatant at the 4th day. |
| Chemical stability | Not realized |

A short clinical assay has been performed on rats to which glioma cells where implanted and on 11 patients with adavanced glioblastomas or astrocytomas, at a dosis ranging from 0.05 to 3.2mg per patient. A significant reduction of the total tumoral volume was observed.

Exemple I

Single stereotactic cerebral intra-ventricular injection in rats implanted with glioma C6 Cells, of 7-β-hydroxy 3-β-oleyl cholesterol (7-OHCE)

- C6 Glioblastoma cells were supplied by the NIH
- Surgery and glioma cell implantation was done according to the WERTHLE and al. procedure.
- Liposomal 7-OHCE was injected 3 days after implantation of C6 Cells.
- The animals were sacrified 10 days after treatment with 7-OHCE.
- Brains were fixed, incubated in para-formaldehyde, soaked in butanol before microtomal serial section was perfomed.
- Total tumoral volume were evaluated after appropriate staining.
- Treatment with 7-OHCE significantly reduced total tumoral volume from a mean value of 4.4mm3 to 0.8mm3.
- A similar experiment was done using "empty" liposomes vs 7-OHCE loaded liposomes.
- **Preliminary Conclusion :**
The injection of liposomal 7-OHCE did affect neither survival nor body weight in experimental animals, while **total glioma cell volume in treated animals were dramatically reduced**.

Exemple II

**Clinical data** "Compassionate" clinical trial.

- 11 patients in total have been included in the first clinical trial (compassionate use).
- Patients included in the study had :

  ➢ Advanced glioblastoma,
  ➢ Grade III o IV astrocytoma,

➢ Anaplastic oligodendroglioma.

■ 7-β-OHCOE was encapsulated in liposomal form and was administed by stereotactic procedure. Patients received from 1 to 3 doses (total amount of 7-β-OHCOE received ranging from 0.05 to 3.2mg/patient).

■ Even though patients included often rank poorly on the KARNOVSKY Scale (end stage patients), partial or total remission was observed in 3 patients 6, 7 and 8). Brain X-Ray pictures are being analysed to assess beyond doubt the observed clinical improvements (I.e. tumor regression).

■ No significant side effects were observed

## Claims

1. Process for producing neutral or charged liposomes containing as the active ingredient 7-β-hydroxy cholesteryl-3-β-oleate (7-β-OHCOE) intended to be, directly or after dissolution in a carrier, administered into malignant gliomas.

2. A process according to claim 1 wherein the liposomes are produced in dissolving 7-β-hydroxy cholesteryl-3-β-oleate in a mixture of dichloromethane and anhydrous ethanol in which previously a soya lecithin and sodium cholesteryl sulphate are dissolved.

3. A process according to claim 1 and claim 2 wherein the liposomes are formed adding a large amount of an aqueous solution buffered at pH 6,8 $\pm$ 0,3 after evaporation of the solvent.

4. A process for manufacturing liposomes of 7-β-hydroxy cholesteryl-3-β-oleate according to claim 1, wherein the evaporation of the mixture of solvents is performed by heating the bath to a temperature of 50-60° C.

5. A process according to any of the preceding claims wherein the liposomes are formed by heating the buffered mixture at 60 - 65 °C to obtain liposomes of homogenous sizes.

6. A process for manufacturing liposomes of 7-β-hydroxy cholesteryl-3-β-oleate according to any of the preceding claims wherein the size of the so produced liposomes ranges from 100 to 600 nm.

7. A process for manufacturing liposomes of 7-β-hydroxy cholesteryl-3-β-oleate according to any of the preceding claims wherein the size of the so produced liposomes ranges from 300 to 400 nm .

8. A process for manufacturing liposomes of 7-β-hydroxy cholesteryl-3-β-oleate according to any of the preceding claims wherein the content of 7 β-hydroxycholesteryl-3-β-oleate in the liposomes range from 1,40 to 1,83 mg/g.

9. A process for manufacturing liposomes of 7-β-hydroxy cholesteryl-3-β-oleate wherein the suspension of liposomes are sterilized by heating at 121°C or by sterilizing filtering.

10. A process for manufacturing liposomes of 7-β-hydroxy cholesteryl-3-β-oleate according to any of the preceding claims wherein the sterilized preparation is poured in flasks of clear glass.

11. Use of the liposomes containing 7-β-hydroxy cholesteryl-3-β-oleate as obtained according to any of the preceding claims, for the treatment of gliomas by direct administration into the brain tumour.

12. Use of the liposomes containing 7-β-hydroxy cholesteryl-3-β-oleate according to claim 11 wherein the administred dosis ranges from 0,05 to 3,2 mg per patient.

13. Use of the liposomes containing 7-β-hydroxy cholesteryl-3-β-oleate according to claim 11 wherein the patients received from 1 to 3 doses of the active ingredient.

Europäisches
Patentamt

European
Patent Office

Office européen
des brevets

# EUROPEAN SEARCH REPORT

Application Number

EP 10 01 0482

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 540 649 A1 (CENTRE NAT RECH SCIENT [FR]) 12 May 1993 (1993-05-12) * claims 3,5,6; examples 6,8,9 * | 1-13 | INV. A61K9/127 A61K31/575 A61P35/00 |
| X | HOR F ET AL: "Therapy of malignant gliomas by oxysterols", STEREOTACTIC AND FUNCTIONAL NEUROSURGERY, S. KARGER AG, vol. 67, no. 1-2, 1 January 1996 (1996-01-01), page 118, XP009143984, ISSN: 1011-6125 * abstract * | 1-13 | |
| X | WERTHLE MYRIAM ET AL: "Local administration of 7-beta-hydroxycholesteryl-3-oleate inhibits growth of experimental rat C6 glioblastoma", CANCER RESEARCH, vol. 54, no. 4, 1994, pages 998-1003, XP002619892, ISSN: 0008-5472 * the whole document * | 1-13 | TECHNICAL FIELDS SEARCHED (IPC) A61K |
| X | BOCHELEN D ET AL: "7beta-Hydroxycholesterol and 7beta-hydroxycholesteryl-3-esters reduce the extent of reactive gliosis caused by an electrolytic lesion in rat brain", NEUROSCIENCE, NEW YORK, NY, US, vol. 51, no. 4, 1 December 1992 (1992-12-01), pages 827-834, XP024385210, ISSN: 0306-4522, DOI: DOI:10.1016/0306-4522(92)90523-5 [retrieved on 1992-12-01] * the whole document * | 1-13 | |

-/--

The present search report has been drawn up for all claims

| Place of search | Date of completion of the search | Examiner |
|---|---|---|
| Munich | 9 February 2011 | Ganschow, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 01 0482

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAKOTOARIVELO C ET AL: "7[beta]-hydroxycholesterol blocked at C-3-OH inhibits growth of rat glioblastoma in vivo: Comparison between 7[beta]-hydroxycholesteryl-3[beta](ester)-oleate and 7[beta]-hydroxycholesteryl-3[beta]-O(ether) oleyl", ANTICANCER RESEARCH 200605 GR, vol. 26, no. 3 A, May 2006 (2006-05), pages 2053-2062, XP009143986, ISSN: 0250-7005 * the whole document * | 1-13 | |

TECHNICAL FIELDS
SEARCHED (IPC)

The present search report has been drawn up for all claims

| Place of search | Date of completion of the search | Examiner |
|---|---|---|
| Munich | 9 February 2011 | Ganschow, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 01 0482

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2011

| Patent document cited in search report | | Publication date | Patent family member(s) | | Publication date |
|---|---|---|---|---|---|
| EP 0540649 | A1 | 12-05-1993 | DE | 69130611 D1 | 21-01-1999 |
| | | | DE | 69130611 T2 | 26-08-1999 |
| | | | FR | 2665179 A1 | 31-01-1992 |
| | | | WO | 9201803 A1 | 06-02-1992 |
| | | | JP | 3133327 B2 | 05-02-2001 |
| | | | JP | 6500015 T | 06-01-1994 |

EPO FORM P0459

For more details about this annex : see Official Journal of the European Patent Office, No. 12/82